# FASCICULE DE BREVET EUROPEEN

(11) **EP 1 537 215 B1**
(45) Date de publication et mention de la délivrance du brevet: **07.06.2006**
(21) Numéro de dépôt: 03769537.6
(22) Date de dépôt: 29.08.2003
(51) Int. Cl.: C12N 15/74

(54) **CASSETTES D EXPRESSION PROCARYOTES REGULEES PAR LE ZINC**
PROKARYOTISCHE EXPRESSIONSKASSETTEN, DIE DURCH ZINK REGULIERT WERDEN
ZINC-REGULATED PROKARYOTIC EXPRESSION CASSETTES

(30) Priorité: 30.08.2002 FR 0210805
(43) Date de publication de la demande: 08.06.2005
(73) Titulaire: INSTITUT NATIONAL DE LA RECHERCHE AGRONOMIQUE (INRA), 75007 Paris Cédex 07 (FR)
(72) Inventeur: POQUET, Isabelle, F-75015 Paris (FR); LLULL, Daniel, F-91300 Massy (FR)
(74) Mandataire: Vialle-Presles, Marie José
(86) Numéro de dépôt international: PCT/FR2003/002606
(87) Numéro de publication internationale: WO 2004/020640

(56) Documents cités:
- US-B1- 6 207 146
- DATABASE EMBL [en ligne] 15 avril 1998 (1998-04-15) POQUET I. ET AL.: "Lactococcus lactis putative lipoprotein Nlp3 precursor." Database accession no. U95834 XP002239449
- MIYOSHI A ET AL: "Controlled production of stable heterologous proteins in Lactococcus lactis." APPLIED AND ENVIRONMENTAL MICROBIOLOGY, vol. 68, no. 6, juin 2002 (2002-06), pages 3141-3146, XP002239446 ISSN: 0099-2240
- POQUET I. ET AL.: "An export-specific reporter designed for gram-positive bacteria: Application to Lactococcus lactis." JOURNAL OF BACTERIOLOGY, vol. 180, no. 7, avril 1998 (1998-04), pages 1904-1912, XP002239447 ISSN: 0021-9193 cité dans la demande
- BOLOTIN ALEXANDER ET AL: "The complete genome sequence of the lactic acid bacterium Lactococcus lactis ssp. lactis IL1403." GENOME RESEARCH, vol. 11, no. 5, mai 2001 (2001-05), pages 731-753, XP001109416 ISSN: 1088-9051

### SEQUENCE LISTING

<110> INSTITUT NATIONAL DE LA RECHERCHE AGRONOMIQUE (INRA)
   Isabelle, POQUET
   Daniel, LLULL
<120> CASSETTES D'EXPRESSION PROCARYOTES REGULEES PAR LE ZINC
<130> MJPbv539/116
<150> FR 02 10805
   <151> 2002-08-30
<160> 14
<170> PatentIn version 3.1
<210> 1
   <211> 32
   <212> DNA
   <213> Artificial sequence
<220>
   <223> Séquence consensus du promoteur bactérien pzn
<220>
   <221> misc_feature
   <222> (9)..(9)
   <223> N = A, C, G ou T
<220>
   <221> misc_feature
   <222> (12)..(18)
   <223> N = A, C, G ou T
<220>
   <221> -35_signal
   <222> (19)..(24)
   <223>
<400> 1
   aaaaataang tnnnnnnntt gacattattt tt 32
<210> 2
   <211> 47
   <212> DNA
   <213> Artificial sequence
<220>
   <223> Séquence consensus du promoteur bactérien pzn
<220>
   <221> misc_feature
   <222> (9)..(9)
   <223> N = A, C, G ou T
<220>
   <221> misc_feature
   <222> (12) .. (18)
   <223> N = A, C, G ou T
<220>
   <221> -35_signal
   <222> (19)..(24)
   <223>
<220>
   <221> misc_feature
   <222> (33)..(41)
   <223> N = A, C, G ou T
<220>
   <221> -10_signal
   <222> (42)..(47)
   <223>
<400> 2
   aaaaataang tnnnnnnntt gacattattt ttnnnnnnnn ntataat 47
<210> 3
   <211> 32
   <212> DNA
   <213> Lactoccocus lactis
<220>
   <221> misc_feature
   <222> (9)..(9)
   <223> Y = T ou C
<220>
   <221> -35_signal
   <222> (19)..(24)
   <223>
<400> 3
   aaaaataayg ttaactggtt gacattattt tt 32
<210> 4
   <211> 56
   <212> DNA
   <213> Lactoccocus lactis
<220>
   <221> -35_signal
   <222> (19)..(24)
   <223>
<220>
   <221> -10_signal
   <222> (42)..(47)
   <223>
<400> 4
   aaaaataatg ttaactggtt gacattattt ttactttgct atataattaa ccagta 56
<210> 5
   <211> 57
   <212> DNA
   <213> Lactoccocus lactis
<220>
   <221> -35_signal
   <222> (20).. (25)
   <223>
<220>
   <221> -10_signal
   <222> (43)..(48)
   <223>
<400> 5
   aaaaaataac gttaactggt tgacattatt ttttctttgc tatataatta accagta 57
<210> 6
   <211> 25
   <212> PRT
   <213> Lactoccocus lactis
<400> 6
   Met Lys Lys Ile Asn Leu Ala Leu Leu Thr Leu Ala Thr Leu Met Gly
   1               5                   10                  15
   Val Ser Ser Thr Ala Val Val Phe Ala
               20                  25
<210> 7
   <211> 18
   <212> DNA
   <213> Artificial sequence
<220>
   <223> Amorce PCR
<400> 7
   ctaatgagcg ggcttttt 18
<210> 8
   <211> 35
   <212> DNA
   <213> Artificial sequence
<220>
   <223> Amorce PCR
<400> 8
   gctctagagc gggatccttc atcgaaactc ttcag 35
<210> 9
   <211> 1100
   <212> DNA
   <213> Lactoccocus lactis
<220>
   <221> -35_signal
   <222> (295)..(300)
   <223>
<220>
   <221> -10_signal
   <222> (318)..(323)
   <223>
<220>
   <221> RBS
   <222> (393)..(348)
   <223>
1220>
   <221> misc_feature
   <222> (362)..(362)
   <223> N = A, C, G ou T
<220>
   <221> misc_feature
   <222> (412)..(412)
   <223> N = A, C, G ou T
<220>
   <221> misc_feature
   <222> (445)..(445)
   <223> N = A, C, G ou T
<220>
   <221> RBS
   <222> (780)..(786)
   <223>
<400> 9
   cacaggaaac agctatgacc atgattacgc caagctcgaa attaaccctc actaaaggga     60
   acaaaagctg ggtaccgggc cccccctcga ggtcgacggt atcgatagcc cgcctaatga    120
   gcgggctttt ttttgatatc gaattacccg ggaattcaga tctttgatca aggatctgtc    180
   agctggttca actagcggtg gtcaaactgt tagtaataaa acttattgtt ttgatgttcg    240
   gcttaaggat ggaaggattt ttcaaataaa aaagtaaaaa ataatgttaa ctggttgaca    300
   ttatttttac tttgctatat aattaaccag taaactaatt atggaggaca aaatactatg    360
   antttagcaa atcaaatcga ccagtttctt ggggcaatta tgcagtttgc anaaaacaag    420
   catgaaatat tactcggcga atgcnaaagt aatgttaagc taacaagcac gcaagaacat    480
   atcttaatga ttctagctgc agaggtttcg acaaacgcga gaattgccga gcaactcaag    540
   atttcgccag cagcggtaac taaagctctc aaaaaattac aagagcaaga actgattaaa    600
   tcaagtcggg caacaaatga cgaacgcgta gtcctttgga gcctgacaga aaaagcaatt    660
   ccagttgcta aagaacatgc tgctcatcat gagaaaactc taagtaccta ccaagaatta    720
   ggagacaaat ttactgacga agaacaaaaa gtgataagtc aattcttatc agtacttacg    780
   gaggagtttc gatgaagaaa atattgatgt tatttgctat tccggcagtt ttacttcttg    840
   ctggttgtca aaaaacagca gacaaaccag aagttgtgac aacttttgag ccgatgtatg    900
   aatttacgaa agcgattgtt ggagataagg ttaaaattga aaatattgtt ccggcgaatc    960
   aagaagttca cgaatttgaa ccgagtgcca ttacgaaaaa aatggtagaa aatgcaaaga   1020
   aaattgaagt cgagtttgac aaaggtcaaa gaactgataa atatggacgt ggcttagcgt   1080
   atatttatgc tgatggaaaa                                                1100
<210> 10
   <211> 160
   <212> DNA
   <213> Lactoccocus lactis
<220>
   <221> -35_signal
   <222> (123)..(128)
<220>
   <221> -10_signal
   <222> (146)..(151)
<400> 10
   gatctgtcag ctggttcaac tagcggtggt caaactgtta gtaataaaac ttattgtttt 60
   gatgttcggc ttaaggatgg aaggattttt caaataaaaa agtaaaaaat aatgttaact 120
   ggttgacatt atttttactt tgctatataa ttaaccagta 160
<210> 11
   <211> 18
   <212> DNA
   <213> Artificial sequence
<220>
   <223> Amorce PCR
<400> 11
   caggaaacag ctatgacc 18
<210> 12
   <211> 27
   <212> DNA
   <213> Artificial sequence
<220>
   <223> Amorce PCR
<400> 12
   gttctaagga tccattaact taaggag 27
<210> 13
   <211> 33
   <212> DNA
   <213> Artificial sequence
<220>
   <223> Amorce PCR
<400> 13
   tttgtgatgc atatgcaaat acaacggctg ttg 33
<210> 14
   <211> 27
   <212> PRT
   <213> Artificial sequence
<220>
   <223> Peptide
<400> 14
   Met Lys Lys Ile Asn Leu Ala Leu Leu Thr Leu Ala Thr Leu Met Gly
   1               5                   10                  15
   Val Ser Ser Thr Ala Val Val Phe Ala Tyr Ala
               20                  25
<210> 15
   <211> 27
   <212> DNA
   <213> Artificial sequence
<220>
   <223> Amorce PCR
<400> 15
   cgcggatcct ttgaaaggat attcctc 27
<210> 16
   <211> 28
   <212> DNA
   <213> Artificial sequence
<220>
   <223> Amorce PCR
<400> 16
   cctacgtatt agaaatgaat gttaaagc 28

## Description

L'invention concerne la production de protéines hétérologues chez des bactéries à Gram-positif, notamment des bactéries lactiques.

A coté de leurs usages traditionnels dans l'industrie agro-alimentaire, les bactéries lactiques sont actuellement de plus en plus utilisées en tant que cellules hôtes pour la production de protéines hétérologues d'intérêt. Ces protéines d'intérêt peuvent être de nature très variée, et il est donc souhaitable de disposer du choix le plus large possible d'outils d'expression pour pouvoir optimiser leur production en fonction des spécificités de chacune d'entre elles.

De manière générale, il est nécessaire d'utiliser des promoteurs forts, permettant d'obtenir un niveau d'expression suffisant du gène d'intérêt. Dans certains cas, on peut utiliser des promoteurs constitutifs ; dans d'autres cas (par exemple lorsque le produit du gène d'intérêt est toxique pour la bactérie hôte ou risque d'interférer avec le métabolisme de celle-ci), il est préférable d'utiliser des promoteurs inductibles, permettant de déclencher ou d'arrêter l'expression au moment souhaité.

Bien que les bactéries lactiques possèdent de nombreux gènes dont la transcription est régulée par divers facteurs, on ne dispose à l'heure actuelle que d'un choix relativement restreint de promoteurs inductibles utilisables en pratique pour la construction de cassettes d'expression de gènes d'intérêt (pour revue, cf. DE VOS, Curr. Op. Microbiol., 2, 289-295, 1999). En effet, cette utilisation demande non seulement que les promoteurs concernés soient régulables, mais encore qu'il existe un différentiel d'expression suffisant entre les différents états d'induction ; idéalement, l'expression doit atteindre un niveau élevé en conditions d'induction, et pouvoir être totalement bloquée en conditions de non-induction. En outre, il est nécessaire de pouvoir maîtriser facilement les facteurs intervenant dans la régulation de ces promoteurs.

Lors de travaux précédents, visant à identifier des protéines exportées de *L. lactis* (POQUET et al., J. Bacteriol., 180, 1904-1912, 1998), les Inventeurs ont cloné, en fusion avec le gène rapporteur Δ_{SP}Nuc, un fragment d'ADN génomique de la souche *L. lactis* MG1363, comprenant un gène dénommé à l'époque *nlp3 (New LipoProtein 3*) , dont le produit présente des homologies avec une protéine de *S. pneumoniae* impliquée dans le transport des métaux. La séquence de ce fragment est disponible sur GENBANK sous le numéro U95834.

Les Inventeurs ont également observé que le gène *nlp3* était négativement régulé par des cations métalliques divalents, notamment Zn²⁺ (POQUET *et al.* « Use of a new reporter tool to demonstrate metal regulation of *nlp3,* a gene putatively involved in metal uptake in *Lactococcus lactis* » ; 6th Symposium on Lactic Acid Bacteria, Veldhoven, Pays-Bas, 19-23 septembre 1999).

D'autre part, dans le cadre du séquençage complet du génome de *L*. *lactis* IL1403, le gène *nlp3,* renommé *zitS,* a été identifié en tant que constituant d'un opéron, dénommé *zitRSQP* (BOLOTIN *et al.,* Antonie van Leeuwenhoek, 76, 27-76, 1999; BOLOTIN *et al.,* Genome Res. 11, 731, 2001). Par homologie avec des séquences connues, des fonctions putatives dans le transport du zinc ont été attribuées aux gènes de cet opéron. Ainsi, le produit du gène *zitP* constituerait la perméase du système de transport, les produits du gène *zitS* et du gène *zitQ* assureraient respectivement la liaison avec le substrat et la liaison avec l'ATP, et le produit du gène *zitR,* qui présente des homologies avec la famille de répresseurs transcriptionnels *marR,* serait impliqué dans la régulation du transport du zinc.

Il n'avait jusqu'à présent pas été envisagé d'utiliser le système de régulation de l'opéron *zitRSQP* pour contrôler l'expression de gènes hétérologues. En effet, bien qu'une régulation négative puisse être déclenchée par l'ajout de zinc (POQUET *et al.,* 1999 précité), le niveau d'expression de base observé en l'absence de cette régulation négative n'apparaissait pas suffisant pour permettre une production satisfaisante de protéines d'intérêt. En outre, on ignorait si le répresseur putatif ZitR était effectivement impliqué dans la répression de l'expression de cet opéron, et si d'autres régulateurs, notamment les régulateurs pléiotropes *flp,* décrits comme intervenant dans la régulation du transport du zinc chez *L. lactis* (GOSTICK et al., Mol. Microbiol., 31, 1523-35, 1999 ; SCOTT et al., FEMS Microbiol. Lett., 192, 85-89, 2000), pouvaient également être impliqués, soit en tant que co-répresseurs, soit au contraire en tant qu'activateurs éventuels.

Or, en poursuivant leurs études sur la régulation de l'opéron *zitRSQP,* les Inventeurs ont constaté, en présence de très faibles concentrations en Zn²⁺, un niveau maximal d'expression beaucoup plus fort que ce que l'on pouvait supposer d'après les expérimentations antérieures, et qui permet d'atteindre un facteur d'induction supérieur à 100. En outre, ils ont constaté que l'expression était indépendante des régulateurs *flp,* et pouvait être totalement contrôlée par l'intermédiaire de la protéine ZitR.

L'étude de la structure du promoteur de l'opéron *zitRSQP* de *L*. *lactis* a permis aux Inventeurs de mettre en évidence, outre les éléments classiquement présents dans les promoteurs bactériens, à savoir les boites -35 (TTGACA) et -10 (TATAAT) séparées par 17pb, une séquence en palindrome chevauchant la boîte -35, qui représente très probablement le site de fixation de ZitR.

Les observations rapportées ci-dessus permettent de supposer que la régulation de *zitRSQP* s'effectue selon le mécanisme suivant : le répresseur ZitR peut former avec le Zn²⁺ intracellulaire un complexe présentant une affinité très importante pour le site de fixation chevauchant la boîte -35 ; le complexe ZitR-Zn²⁺ fixé sur le palindrome empêche l'accès de l'ARN polymérase à la boîte -35, et réprime donc la transcription ; en revanche, la forme non-complexée de ZitR ne se fixe pas sur le site -35, permettant la transcription de l'opéron, qui s'effectue alors avec une grande efficacité.

La régulation de l'opéron *zitRSQP* par l'intermédiaire de ZitR dépend donc de la concentration intracellulaire en Zn²⁺, elle-même fonction de la disponibilité en Zn²⁺ dans le milieu de culture.

L'opéron *zitRSQP* représente très probablement un système de transport du zinc de très haute affinité qui n'est utilisé par la bactérie que dans des conditions de carence en zinc très sévère, pour permettre la survie cellulaire ; en revanche, dans les conditions de culture habituelles sur milieux riches, le zinc est présent en abondance dans l'environnement, et transporté dans la cellule par des systèmes de moindre affinité que le complexe ZitSPQ, dont la synthèse est alors réprimée.

Ces propriétés du système de régulation de l'opéron *zitRSQP* de *L. lactis* mises en évidence par les Inventeurs permettent de proposer son utilisation pour la production de protéines d'intérêt dans des bactéries-hôtes, notamment des bactéries Gram-positif, et en particulier des bactéries lactiques.

La présente invention a pour objet les différents aspects de cette utilisation.

Selon une première variante, la présente invention a pour objet un procédé de construction d'une cassette d'expression d'une séquence nucléotidique d'intérêt, caractérisé en ce que l'on utilise pour contrôler l'expression de ladite séquence d'intérêt, un polynucléotide constitué par :
- un promoteur bactérien, dénommé ci-après p_{Zn}, contenant un site de fixation pour la protéine ZitR de *Lactococcus lactis,* lequel site comprend la séquence suivante :
   AAAAATAANGTNNNNNNNTTGACATTATTTTT (SEQ ID NO:1),
dans laquelle TTGACA représente la boîte -35 dudit promoteur, et N représente A, C, G, ou T ;
- une séquence codant pour un polypeptide présentant au moins 80%, de préférence au moins 85%, et de manière tout à fait préférée au moins 95% d'identité avec la protéine ZitR de *Lactococcus lactis* placée sous contrôle transcriptionnel dudit promoteur ;
- et en ce que l'on associe ledit polynucléotide avec au moins un site de restriction permettant l'insertion d'une séquence nucléotidique d'intérêt sous contrôle transcriptionnel dudit promoteur.

Selon un mode de réalisation préféré de la présente invention, le promoteur p_{Zn} comprend la séquence suivante :
AAAAATAANGTNNNNNNNTTGACATTATTTTTNNNNNNNNNTATAAT(SEQ ID NO:2)
dans laquelle TATAAT représente la boîte -10 dudit promoteur.

Selon un autre mode de réalisation préféré de la présente invention, ledit promoteur p_{Zn} contient un site de fixation pour la protéine ZitR de *Lactococcus lactis* comprenant la séquence suivante :
AAAAATAAYGTTAACTGGTTGACATTATTTTT (SEQ ID NO:3),
dans laquelle Y représente T ou C.
A titre d'exemple de promoteurs p_{Zn} utilisables pour la construction d'une cassette d'expression conformément à l'invention, on citera :
- le promoteur p_{Zn} de la souche MG1363 de *Lactococcus lactis,* qui comprend la séquence:
   AAAAATAATGTTAACTGGTTGACATTATTTTTACTTTGCTATATAATTAACCAGTA (SEQ ID NO:4) ;
- le promoteur p_{Zn} de la souche IL1403 de *Lactococcus lactis,* qui comprend la séquence :
   AAAAAATAACGTTAACTGGTTGACATTATTTTTTCTTTGCTATATAATTAACCAGTA (SEQ ID NO:5)

Selon une seconde variante, la présente invention a pour objet un procédé de construction d'une cassette d'expression d'une séquence nucléotidique d'intérêt, caractérisé en ce que l'on utilise pour contrôler l'expression de ladite séquence d'intérêt, un polynucléotide constitué par :
- un promoteur bactérien p_{Zn}, tel que défini ci-dessus ;
- et en ce que l'on associe ledit polynucléotide avec au moins un site de restriction permettant l'insertion d'une séquence nucléotidique d'intérêt sous contrôle transcriptionnel dudit promoteur.

Selon un mode de réalisation préféré de la présente invention, on effectue l'insertion d'une séquence nucléotidique d'intérêt dans une cassette d'expression obtenue par un procédé conforme à la première ou à la seconde variante de l'invention, sous contrôle transcriptionnel du promoteur p_{Zn}.

Ladite séquence nucléotidique d'intérêt peut être toute séquence que l'on souhaite exprimer sous contrôle transcriptionnel du promoteur p_{Zn}. Il peut s'agir notamment de toute séquence codant pour une protéine hétérologue d'intérêt que l'on désire produire dans une bactérie-hôte ; ladite protéine peut le cas échéant être une protéine de fusion, associant des séquences polypeptidiques d'origine diverse.

Des cassettes d'expression obtenues par un procédé conforme à l'invention peuvent, le cas échéant, comprendre en outre les éléments nécessaires pour permettre l'adressage de la protéine d'intérêt à la surface cellulaire, ou sa sécrétion dans le milieu de culture.

Dans ce cadre, selon un mode de réalisation préféré de la présente invention on effectue l'insertion d'une séquence nucléotidique codant pour un peptide d'adressage extracellulaire, et d'au moins un site de restriction permettant le clonage d'une séquence nucléotidique d'intérêt en fusion traductionnelle avec ledit peptide d'adressage, sous contrôle transcriptionnel du promoteur p_{Zn}, dans une cassette d'expression obtenue par un procédé conforme à l'invention.

Ledit peptide d'adressage peut être par exemple un peptide signal de sécrétion, un domaine transmembranaire, un signal d'ancrage à la paroi, etc.

De nombreux peptides d'adressage utilisables dans le cadre de la présente invention sont connus en eux mêmes. A titre d'exemples non limitatifs, on citera les peptides décrits dans la publication de POQUET et al. (1998, précité), ou dans la publication de LE LOIR et al. (Appl. Environ. Microbiol., 67, 4119-4127, 2001).

Pour la production de protéines sécrétées, un peptide d'adressage extracellulaire préféré est le peptide signal de la protéine Exp4 de *L. lactis,* qui répond à la séquence :
MKKINLALLTLATLMGVSSTAVVFA (SEQ ID NO: 6).

La présente invention a également pour objet un procédé de construction d'un vecteur recombinant comprenant la construction d'une cassette d'expression par un procédé conforme à l'invention, et l'insertion de ladite cassette dans un vecteur. La présente invention a également pour objet un procédé de transformation d'une bactérie à gram-positif comprenant la construction d'une cassette d'expression par un procédé conforme à l'invention et la transformation de ladite bactérie par ladite cassette d'expression.

De préférence, ladite bactérie est choisie parmi les bactéries lactiques, notamment des lactocoques, des lactobacilles ou des streptocoques thermophiles.

Le cas échéant, il peut s'agir de bactéries provenant de souches bactériennes comportant une ou plusieurs modifications de leur génome, visant à améliorer la production et/ou la sécrétion de protéines exprimées dans lesdites bactéries, et/ou à éviter leur dégradation. Par exemple, dans le cadre de la production de protéines exportées, on peut utiliser une souche bactérienne dans laquelle l'activité protéasique PrtP et/ou l'activité protéasique HuA sont inactives, telle que celle décrite dans la Demande PCT WO 00/39309 ou une souche bactérienne surproduisant une protéine permettant de stabiliser les protéines exportées, telle que la protéine Nlp4 de *Lactococcus lactis,* ou un de ses homologues (POQUET et al. 1998, publication précitée).

La présente invention a également pour objet l'utilisation de cassettes d'expression ou de vecteurs recombinants susceptibles d'être obtenus par un procédé conforme à l'invention pour la production de protéines d'intérêt dans une bactérie à gram-positif, notamment des bactéries lactiques.

Les cassettes d'expression susceptibles d'être obtenues par un procédé conforme à la première variante de l'invention peuvent être utilisées dans une bactérie-hôte, pour contrôler le moment de l'expression d'un gène d'intérêt inséré dans le site de clonage, et le niveau de cette expression.

Lorsque l'on cultive la bactérie-hôte en présence d'une quantité de zinc en excès par rapport à ses besoins, l'expression du gène d'intérêt est totalement réprimée. La déplétion en Zn²⁺ du milieu de culture (qui peut s'effectuer simplement par addition d'un agent chélateur des cations divalents, tel que l'EDTA) permet de lever la répression, et de provoquer l'expression du gène. Le niveau d'expression peut être facilement régulé par la quantité d'agent chélateur ajouté.

On peut aussi mettre en culture la bactérie-hôte dans un milieu comprenant une quantité de zinc juste suffisante pour couvrir ses besoins pendant une période donnée de la culture (par exemple pendant la phase de croissance). Dans ce cas, l'épuisement du zinc à l'issue de cette période provoque l'expression du gène d'intérêt.

Dans ce cadre, la présente invention a pour objet un procédé de production d'une protéine d'intérêt dans une bactérie à gram-positif, et notamment une bactérie lactique, caractérisé en ce qu'il comprend :
- l'introduction dans ladite bactérie d'une cassette d'expression susceptible d'être obtenue par un procédé conforme à la première variante de l'invention, comprenant une séquence codant pour ladite protéine d'intérêt ;
- la culture de ladite bactérie dans un milieu contenant une quantité de Zn²⁺ suffisante pour réprimer l'expression de ladite protéine ;
- l'induction de la production de ladite protéine par déplétion en Zn²⁺dudit milieu ;
- la récupération de la protéine produite.

Selon un mode de mise en oeuvre préféré du procédé conforme à l'invention, la déplétion en Zn²⁺ dudit milieu est obtenue par addition d'un chélateur des cations divalents.

Selon un autre mode de mise en oeuvre préféré de la présente invention, la déplétion en Zn²⁺ dudit milieu est obtenue par culture de la bactérie jusqu'à épuisement du Zn²⁺ initialement présent dans le milieu.

D'après les expérimentations effectuées par les Inventeurs sur la souche modèle MG1363 de *L. lactis,* le maintien pendant toute la durée de la culture d'une quantité de Zn²⁺ suffisante pour réprimer l'expression sous contrôle du système p_{Zn}/ZitR peut être assuré par l'utilisation d'un milieu contenant en début de culture, de l'ordre de 1 à 2 µM de Zn²⁺, le seuil de répression totale pouvant être estimé à entre 100nM et 1µM de Zn²⁺. La concentration en Zn²⁺ du milieu en dessous de laquelle on obtient la levée complète de la répression de p_{zn} est très faible (de l'ordre du nanomolaire, et au maximum de quelques nanomolaires). La quantité de chélateur des cations divalents nécessaire pour effectuer la déplétion en Zn²⁺ et induire l'expression sous contrôle du promoteur p_{Zn} varie selon la quantité de Zn²⁺ apportée initialement au milieu de culture ; à titre indicatif, dans le cas de la souche MG1363, pour un milieu de culture riche tel que le milieu M17, une déplétion en Zn²⁺ permettant d'induire une expression maximale peut être obtenue à partir d'une concentration d'EDTA de l'ordre de 0,1 mM ; en milieu SA, qui contient 10nM de Zn²⁺ une déplétion en Zn²⁺ permettant d'induire une expression maximale peut être obtenue à partir d'une concentration d'EDTA de l'ordre de 0,01 mM.

Les quantités de Zn²⁺ et d'agent chélateur de cations mentionnées ci-dessus sont données à titre indicatif. A partir de ces indications, et des autres informations fournies par le descriptif de la présente invention, l'homme du métier peut facilement déterminer, par des tests préalables, effectués par exemple en plaçant un gène rapporteur sous contrôle du système p_{zn}/ZitR dans une cassette d'expression conforme à l'invention, les quantités les plus appropriées selon l'espèce ou la souche bactérienne concernée, les conditions opératoires mises en oeuvre, telles que le milieu utilisé, les modalités de l'ajout de Zn²⁺ et/ou d'agent chélateur (par exemple, en une seule fois, en plusieurs fois, en continu, etc.), et le niveau d'expression souhaité.

Des cassettes d'expression susceptibles d'être obtenues par un procédé conforme à la seconde variante de l'invention seront utilisées de préférence dans des souches de bactéries, notamment de lactocoques, dans lesquelles le répresseur ZitR endogène est inactif, ainsi qu'éventuellement le complexe ZitSPQ. Dans ces conditions, le promoteur p_{Zn} constitue un promoteur fort, permettant l'expression de la protéine d'intérêt pendant toute la durée de la culture. L'inactivation du récepteur ZitR et du complexe ZitSPQ pent s'effectuer de manière connue en elle-même, notamment par mutagenèse dirigée de l'opéron *zitRSQP.*

Dans ce cadre, la présente invention a pour objet un procédé de production d'une protéine d'intérêt dans une bactérie à gram-positif dans laquelle le répresseur ZitR endogène est inactif, caractérisé en ce qu'il comprend :
- l'introduction dans ladite bactérie d'une cassette d'expression susceptible d'être obtenue par un procédé conforme à la seconde variante de l'invention, comprenant une séquence codant pour ladite protéine d'intérêt ;
- la culture de ladite bactérie ;
- la récupération de la protéine produite.

La présente invention peut être mise en oeuvre par exemple :
- dans le domaine de la production de protéines hétérologues d'intérêt thérapeutique par génie génétique, pour mieux contrôler la production de ces protéines par les cultures de bactéries transformées ;
- en industrie agro-alimentaire, notamment dans la fabrication de produits fermentés, pour contrôler selon le stade de la fermentation, la production de protéines d'intérêt permettant notamment d'influer sur la qualité du produit fermenté fini.

La présente invention sera mieux comprise à l'aide du complément de description qui va suivre, qui se réfère à des exemples non-limitatifs, illustrant la construction de cassettes d'expression conformément à l'invention.

### EXEMPLE 1 : REGULATION DE ZIT PAR ZN²⁺ CHEZ L. LACTIS

L'expression de *zit* en fonction de la concentration en Zn²⁺ du milieu est mesurée par 2 techniques différentes:
*La mesure de l'activité Nuc de la fusion ZitRS-Δ_{SP}Nuc, portée par le plasmide pVE8020, dans la souche de *L. lactis* MG1363. Le plasmide pVE8020 résulte du clonage du fragment d'ADN chromosomique de la souche MG1363 correspondant à p_{Zn}*zitRzitS'* (GenBank U95834) dans le plasmide pFUN (POQUET et al., 1998, précité ; GenBank AF038666).
*La quantification de l'ARNm de *zitS* dans la souche MG1363 sauvage (expression endogène de *zitS)* et dans la souche MG1363 transformée par pVE8020).

### Effet du zinc sur l'activité Nuc sous contrôle du promoteur P_{Zn}

Deux types d'expériences ont été faites :

1). La mesure de l'activité Nuc (nucléase de *Staphylococcus aureus)* a été effectuée sur des boîtes de culture contenant du milieu chimiquement défini SA (JENSEN et HAMMER, *Appl. Env. Microbiol.* 59, 4363-66, 1993), qui comprend une quantité minimale de chacun des éléments nécessaires à la croissance bactérienne, et est notamment pauvre en zinc (10 nM de ZnSO₄).

On effectue sur ce milieu un dépôt d'une solution de Zn²⁺ (20 µl de ZnSO₄ à 0,1M) sous forme d'une strie traversant la boîte.

Après absorption du dépôt de Zn²⁺, on effectue 2 dépôts de bactéries sous forme de 2 stries parallèles entre elles, et coupant perpendiculairement la strie de zinc : un dépôt témoin, (souche MG1363 transformée par un plasmide (pVE8009) portant la fusion Usp-Δ_{SP}Nuc sous contrôle du promoteur Usp) et un dépôt de la souche MG1363 transformée par pVE8020 (MG1363(pVE8020)).

L'incubation pendant une nuit à 30°C des boîtes permet la croissance des bactéries et la création d'un gradient de concentrations en Zn²⁺ décroissantes par diffusion à partir de la strie de ZnSO₄.

On réalise un test coloré de l'activité nucléasique, en déposant sur les boîtes une surcouche de détection contenant du bleu de toluidine et en incubant à 37°C (LACHICA et al., *Appl. Microbiol.,* 21, 585-87, 1971; LE LOIR et al., *J. Bacteriol.,* 176, 5135-39, 1994) : l'activité Nuc est détectée par le virage au rose de la surcouche de détection, formant un halo autour des stries de dépôts bactériens.

Alors que le halo observé autour de la strie correspondant au dépôt témoin est de taille et d'intensité constante sur toute la longueur de la strie bactérienne (ce qui indique que ni l'activité nucléasique d'Usp-Δ_{SP}Nuc, ni son exportation, ni son expression ne dépendent du Zn²⁺), celui observé autour de la strie correspondant au dépôt MG1363(pVE8020) est limité à l'extrémité la plus éloignée du dépôt de zinc, où sa largeur et son intensité sont comparables à celles observées pour le dépôt témoin ; l'intensité décroît à l'approche du dépôt de zinc, et on n'observe aucun halo au voisinage de l'intersection avec celui-ci.

Il apparaît donc que des concentrations élevées de zinc répriment l'expression du promoteur de l'opéron *zitRSQP.*

2) La régulation a aussi été étudiée par détection du rapporteur Nuc sur un gel SDS-PAGE grâce à son activité enzymatique (zymogramme). Pour cette expérience, le milieu SA a été encore appauvri en zinc, soit en omettant toute addition de ZnSO₄ lors de sa préparation, soit en lui ajoutant de l'EDTA à 0,0 1nM. La souche de *L. lactis* MG1363 (pVE8020) a été inoculée dans ce milieu, puis la culture a été divisée en deux pour subir ou non une addition de 2µM de Zn²⁺. Après croissance à 30°C sans agitation pendant la nuit, des échantillons de culture ont été prélevés en normalisant leur volume en fonction de leur DO₆₀₀ de façon à obtenir un nombre de cellules équivalent à celui de 1 ml de culture à DO₆₀₀ = 1. Les échantillons ont été précipités par l'acide tri-chloroacétique concentré, lavés, lysés en présence de lysozyme et de SDS, et repris dans un tampon de chargement, selon le protocole décrit dans POQUET *et al.* (1998, précité).

Les protéines ont ensuite été séparées selon leur poids moléculaire sur un gel SDS-PAGE à 12,5% d'acrylamide. L'activité Nuc été détectée comme décrit ci-dessus. Trois formes protéiques présentant une activité nucléasique (qui d'après leur poids moléculaire, correspondent au précurseur au peptide-signal non clivé, à la forme mature Nlp3-Δ_{SP}Nuc, et au produit de dégradation NucA) ont été détectées uniquement dans l'échantillon de culture n'ayant subi aucune addition. Aucune protéine possédant une activité Nuc n'a été détectée dans l'échantillon supplémenté en Zn²⁺. Ces résultats démontrent que la répression est totale en présence de 2µM de Zn²⁺.

### Effet de l'EDTA sur l'activité Nuc sous contrôle du promoteur p_{Zn}

La mesure de l'activité Nuc est effectuée sur des boites de culture contenant du milieu M17 (TERZAGHI and SANDINE, *Appl. Environ. Microbiol., 29,* 807-13, 1975) riche en zinc.

On effectue un dépôt d'une solution d'EDTA (20 µl à 0,1M), ainsi qu'un dépôt de la souche MG1363 contenant le plasmide témoin, et un dépôt de la souche MG1363(pVE8020) sous forme de stries, comme décrit ci-dessus.

Après incubation à 30°C pendant une nuit, l'activité Nuc est détectée comme décrit ci-dessus.

Le halo observé autour de la strie correspondant au dépôt témoin est, comme celui observé dans le cas du zinc, de taille et d'intensité constante sur toute la longueur de la strie. En revanche, de façon surprenante, celui observé autour de la strie correspondant au dépôt MG1363(pVE8020) est, au voisinage du dépôt d'EDTA, beaucoup plus intense que celui du dépôt témoin ; l'intensité décroît très rapidement lorsque l'on s'éloigne de cette intersection.

Il apparaît donc que l'EDTA induit l'expression du promoteur de l'opéron *zitRSQP.* Le niveau d'expression apparaît en outre plus élevé que celui observé à distance de la strie de zinc dans l'expérimentation précédente, et en outre plus élevé que celui du témoin, contrôlé par le promoteur Usp : on peut donc atteindre un très fort niveau d'induction de p_{Zn} *zitR* grâce à des concentrations en EDTA qui n'affectent pas la croissance bactérienne (comme 0,1mM en M17).

### Evaluation quantitative de l'effet de l'EDTA sur l'activité Nuc sous contrôle du promoteur p_{Zn}

Pour évaluer quantitativement l'effet de l'EDTA sur l'activité Nuc exprimée sous contrôle du promoteur de l'opéron *zitRSQP,* les expérimentations suivantes sont effectuées :

La souche MG1363 transformée par pVE8020 est cultivée en milieu M17 supplémenté en érythromycine à 5 µg/l, jusqu'en phase exponentielle (DO₆₀₀ = 0,3) ou stationnaire (D0₆₀₀ = 1,2). Cette culture est divisée en 4 sous-cultures ; 3 d'entre elles sont alors additionnées d'EDTA à diverses concentrations finales (3,3 mM ; 0,33 mM; 0,033 mM); la quatrième ne reçoit pas d'addition d'EDTA (0 mM EDTA). Après 30 min ou 1h30 d'incubation, des échantillons de chaque culture sont prélevés. Le nombre de cellules de chaque échantillon est normalisé en adaptant le volume de façon à obtenir un nombre de cellules équivalent à celui celui de 1 ml de culture à DO₆₀₀ = 1.

Les cellules sont ensuite lysées et précipitées par un traitement à l'acide tri-chloroacétique concentré (16,7%), lavées à l'acétone (80%) et reprises dans 100 µl de tampon Tris (50 mM pH7). 10µl de chaque échantillon ainsi traité sont déposés sur une boîte contenant du milieu de détection de l'activité Nuc (LACHICA et al., 1971 ; LE LOIR et al., 1994, précité). L'activité Nuc est évaluée par la taille du halo et l'intensité de la coloration rose autour de chaque dépôt. Pour une évaluation quantitative du niveau d'activité, on dépose sur la même boîte une gamme étalon de protéine Nuc purifiée (dilution de 4 en 4 à partir de 400 ng).

On constate que la taille du halo et l'intensité de la coloration varient en fonction de la concentration en EDTA qui a été utilisée pour traiter les cellules. En l'absence d'EDTA, on observe un halo très fin sans coloration nette ; à 0,033 mM d'EDTA, on observe un fin halo, nettement coloré en rose ; à 0,33 mM, on observe un halo large, de coloration rose très intense. On n'observe aucune augmentation de la taille et de l'intensité du halo de 0,33 à 3,3 mM. Aucune différence significative de la taille et de l'intensité du halo n'est notée entre l'addition d'EDTA effectuée en phase exponentielle et celle effectuée en phase stationnaire, ni entre les deux temps d'incubation (30 mn ou 1h 30).

Ces résultats indiquent que le niveau d'induction de l'expression de p_{Zn} augmente avec la concentration en EDTA ajoutée, jusqu'à un seuil de saturation (qui est atteint en milieu M17, dans les conditions expérimentales décrites ici, pour une concentration en EDTA de l'ordre de 0,33 mM, quelle que soit la phase de croissance et le temps d'incubation).

La comparaison avec la gamme étalon de protéine Nuc purifiée permet d'estimer que le niveau d'induction obtenu par addition de 0,33 mM d'EDTA en milieu M17 est de l'ordre de 100.

### Effet du zinc sur la transcription de l'opéron zit

Les souches utilisées sont la souche sauvage de *L. lactis* subsp *cremoris* MG1363, et sa dérivée mutante FNR (double mutant *flpA flpB,* SCOTT et al., 2000, précité ; GOSTICK et al., 1999, précité). Les gènes *flp* sont des régulateurs pléiotropes intervenant notamment dans le transport du zinc : chez FNR, la concentration intracellulaire en zinc est sept à huit fois plus faible que celle de la souche sauvage (GOSTICK et al., 1999, précité).

A partir d'une préculture d'une nuit de chaque souche en milieu SA supplémenté pour FNR uniquement, en érythromycine à 5 µg/µl et en tétracycline à 5 µg/µl, on effectue une culture à 30°C en milieu SA (dont la concentration en Zn²⁺ est de 10nM). En phase exponentielle précoce (DO₆₀₀ 0,07 à 0,08), cette culture est divisée en deux parties : l'une (+) est additionnée de ZnSO₄ pour obtenir une concentration finale en Zn²⁺ de 2 µM (qui n'affecte pas la croissance) ; l'autre (-) ne reçoit aucune addition. La culture est poursuivie sans modification jusqu'en phase exponentielle (DO₆₀₀ = 0,2) ou stationnaire (DO₆₀₀ = 0,8). L'ARN des bactéries est alors extrait selon le protocole décrit par RAYA et al., *(J. Bacteriol.,* 180, 3174-80, 1998).

Après l'extraction, la concentration en ARN est évaluée par mesure de la D0₂₆₀: 60 µg d'ARN sont déposés sur un gel à 1% d'agarose. Après migration et transfert sur une membrane de nylon, les transcrits *zitRS* sont détectés par transfert de Northern, avec une sonde spécifique du gène *zitS.*

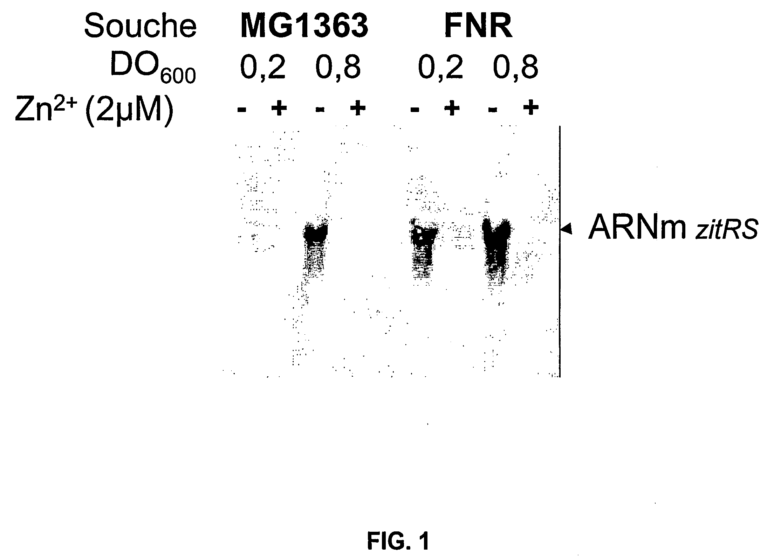

Les résultats sont illustrés par la Figure 1.

Ces résultats montrent que l'on n'observe un ARNm spécifique de la taille attendue pour *zitRS* qu'en l'absence (-) d'addition de Zn²⁺ et jamais en sa présence (+) quelle que soit la souche, et quelle que soit la phase de croissance au moment de l'addition.

Cela montre 1) que la répression par le Zn²⁺ de l'expression de l'opéron *zit* s'effectue au niveau transcriptionnel, 2) qu'elle est totale pour une concentration en Zn²⁺ du milieu de 2µM, et 3) qu'elle est indépendante des gènes *flp*, puisqu'elle s'exerce dans le mutant FNR. Ce dernier point indique que la régulation par le Zn²⁺ dépend entièrement du régulateur *zitR.*

En absence d'addition de Zn²⁺, on observe un niveau d'expression très fort, sauf pour la souche MG1363 en phase exponentielle, où l'on observe qu'une faible expression. Ces résultats indiquent que malgré la très faible concentration en Zn²⁺ (10 nM) du milieu SA de départ, la concentration intracellulaire en Zn²⁺ au moment de la phase exponentielle où a été effectué le prélèvement est encore suffisante, dans le cas de la souche MG1363, pour réprimer fortement la transcription de l'opéron *zit*. En revanche, en phase stationnaire, après épuisement du Zn²⁺ présent dans le milieu, l'expression est très forte. Dans le cas de la souche FNR, la concentration en Zn²⁺ de 10 nM du milieu de départ est insuffisante pour assurer, même pendant la phase exponentielle, une concentration en Zn²⁺ intracellulaire réprimant la transcription de l'opéron *zit.*

Il apparaît donc que l'induction de l'expression dépend directement de la concentration intracellulaire en Zn²⁺, et que celle-ci doit être très faible pour obtenir une expression maximale.

Ceci peut être obtenu notatnment :
1) En abaissant la concentration extra-cellulaire en Zn²⁺ ; celle-ci doit en effet être très inférieure à 10nM, où l'on observe encore une répression importante par rapport au niveau d'induction maximal. L'abaissement de la concentration extra-cellulaire en Zn²⁺ peut par exemple s'effectuer par l'addition d'un agent chélateur comme l'EDTA (quelle que soit la phase de croissance), ou en opérant en phase stationnaire, dans des conditions où le Zn²⁺ initialement présent dans le milieu a été consommé par les bactéries au cours de la croissance, ou par une combinaison de ces deux moyens.
2) En utilisant des mutants dans lesquels le transport du zinc est affecté, et qui possèdent de ce fait une concentration en Zn²⁺ intra-cellulaire très faible, tels que la souche FNR mentionnée ci-dessus.

### EXEMPLE 2: CONSTRUCTION DE VECTEURS D'EXPRESSION SOUS CONTROLE DU SYSTEME DE REGULATION DE L'OPERON ZITRSQP

### Construction de plasmides contenant le système de régulation de l'opéron zitRSQP

### Plasmide pDI11

Le système promoteur-régulateur p_{Zn}-*zitR* de la souche MG1363 est obtenu par amplification PCR (kit DyNAzyme EXT de FINNZYMES) d'une partie de l'insert p_{Zn}*zitRzitS'* (GenBank U95834) du plasmide pVE8020 avec les oligonucléotides oligo 9 et oligo MUT :
Oligo 9 :
5'-CTAATGAGCGGGCTTTTT-3' (SEQ ID NO: 7)
Oligo MUT :
5'-GCTCTAGAGCGGGATCCTTCATCGAAACTCTTCAG-3'(SEQ ID NO: 8)

Oligo 9 s'hybride avec le site de clonage multiple (MCS) de pFUN, et permet d'amplifier tout insert cloné dans ce vecteur. Oligo MUT permet d'éliminer le site de fixation potentiel des ribosomes (RBS) de *zitS* et pour faciliter le clonage du fragment PCR : sa séquence, située dans la zone de chevauchement entre *zitR* et *zitS,* présente deux mutations (soulignées) dans le RBS (la séquence sauvage 5'-GGAGGAG-3' est mutée en 5'-TGAAGAG-3', complémentaire de 5'-CTCTTCA-3' dans l'oligo MUT) et les deux sites de restriction *Bam*HI et *Xba*I (en gras).

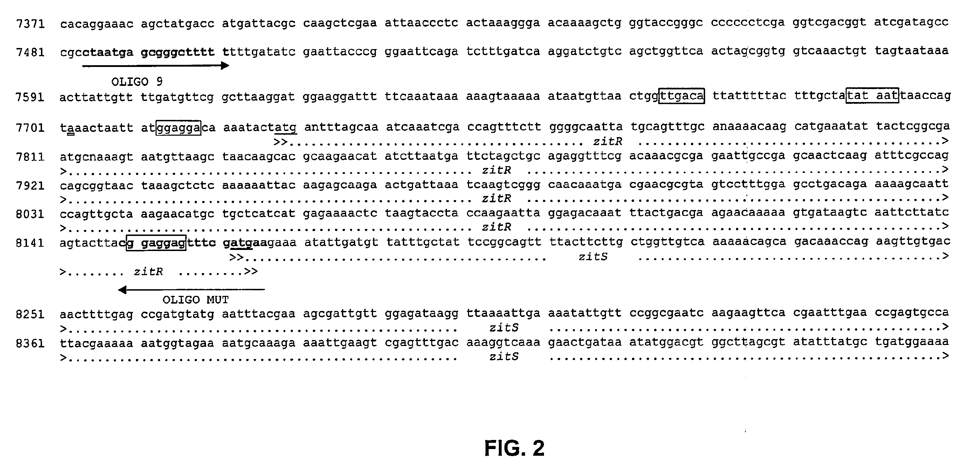

La séquence de la région du plasmide pVE8020 à partir de laquelle est effectuée l'amplification (SEQ ID NO: 9) est représentée sur la Figure 2. Les chiffres à gauche de la séquence correspondent à la numérotation de la séquence entière du plasmide pVE8020. Les zones d'appariement des amorces oligo 9 et oligo MUT sont représentées en gras et avec des flèches. Les séquences codant pour ZitR et une partie de ZitS sont indiquées. Les RBS (sites de fixation des ribosomes) potentiels de *zitR* et *zitS* sont encadrés, et les codons d'initiation de la traduction ATG sont soulignés. Les boites -35 et -10 du promoteur sont encadrées et surlignées en gris ; le site potentiel d'initiation de la transcription est indiqué par un soulignement double.

Le produit d'amplification de 700 pb est traité par le fragment Klenow (PolIK) de l'ADN polymérase d'*Escherichia coli,* puis par *Xba*I*.* Ce fragment modifié est purifié et cloné dans le vecteur pFUN, préalablement digéré par *Eco*RV et *Xba*I : le mélange de ligation (fragment + pFUN + ligase du phage T4) est utilisé pour électroporer la souche MG1363 de *Lactococcus lactis,* et des clones résistant à l'érythromycine sont sélectionnés sur milieu solide M17 + glucose 0,5% + érythromycine 5 µg/mL. Un de ces clones, contenant un plasmide recombinant de 8,2 kb, dénommé ci-après pDI11, est choisi.

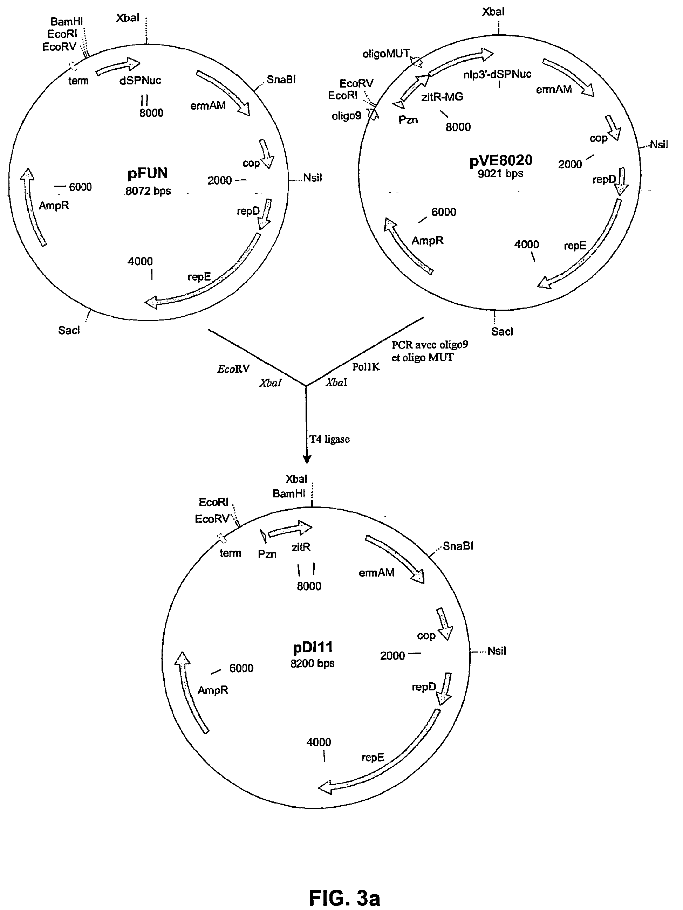

Les étapes de la construction de ce plasmide sont représentées sur la Figure 3a.

Il contient la totalité de la séquence codant pour ZitR, ainsi qu'une séquence 5' comprenant le promoteur P_{Zn}. Cette séquence 5' (SEQ ID NO:10) est représentée ci-après (jusqu'au site potentiel d'initiation de la transcription):
GATCTGTCAGCTGGTTCAACTAGCGGTGGTCAAACTGTTAGTAATAAAACTTATTGT TTTGATGTTCGGCTTAAGGATGGAAGGATTTTTCAAATAAAAAAGTAAAAAATAATG TTAACTGGTTGACATTATTTTTACTTTGCTATATAATTAACCAGTA

### Plasmide pDI12

pDI11 est digéré par *Eco*RI et *Eco*RV et traité par PolIK pour obtenir un fragment linéaire de 8,18 kb dépourvu des sites de restriction du MCS de pFUN (ce qui permet de les introduire ultérieurement ailleurs dans la construction), puis traité par la ligase du phage T4. La souche MG1363 est électroporée par le mélange de ligation, et un clone résistant à l'érythromycine et contenant le plasmide pDI12 est sélectionné comme décrit ci-dessus.

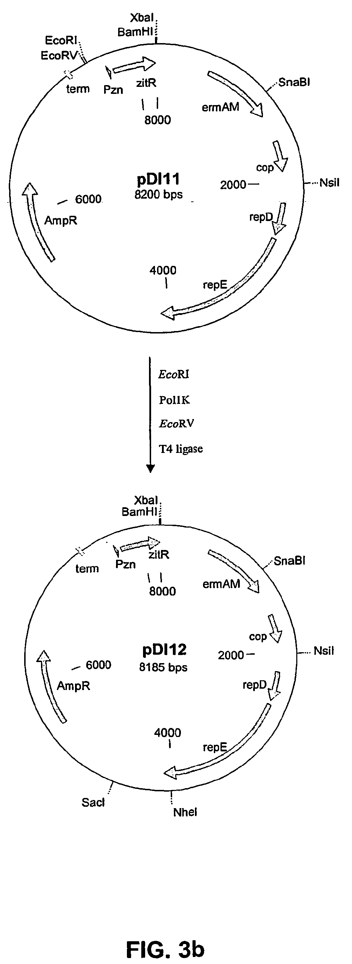

Les étapes de la construction de ce plasmide sont représentées sur la Figure 3b.

### Construction de plasmides contenant un gène rapporteur sous contrôle du système de régulation de l'opéron zitRSQP

### Plasmide pDI24

pDI24 comprend les éléments suivants : une séquence codant pour une protéine rapporteur permettant de tester le système, suivie par un terminateur.

La protéine rapporteur choisie est NucB, la forme dépourvue de peptide signal de la nucléase Nuc de *Staphylococcus aureus* (SHORTLE, Gene, 22, 181-189, 1983). Son cadre ouvert de lecture est cloné dans le plasmide pSEC1 (ou pVE3684, CHATEL *et al,* Clin. Diagn. Lab. Immunol., 8, 545-551, 2001) sous le contrôle du promoteur Pₙᵢₛ (inductible par la nisine) pour la transcription, et des signaux d'Usp45 de *L. lactis* pour la traduction (RBSUsp45 et codon d'initiation) et la sécrétion (peptide signal PSUsp45) : l'ensemble pₙᵢₛ-RBSUsp45-PSUsp45 est cloné dans pDI24.

Le terminateur choisi est le terminateur T1T2 (PESCHKE *et al,* J. Mol. Biol., 186, 547-555, 1985) qui provient du plasmide pVE5239 (DIEYE *et al,* J. Bacteriol., 183, 4157-4166, 2001).

Pour la construction de pDI24, pSEC1 est digéré par *Xho*I*,* traité par l'ADN polymérase du phage T4, et digéré par *Cla*I*,* pour obtenir une forme linéaire de 3,8 kb. En parallèle, pVE5239 est digéré par *Sac*I*,* traité par l'ADN polymérase du phage T4, et digéré par *Cla*I*,* pour obtenir un fragment de 217 pb contenant le terminateur T1T2. Ce fragment est purifié et ligaturé avec la forme linéaire du vecteur pSECl, et le mélange de ligation est utilisé pour transformer la souche TG1 d'*E*. *coli.* Des clones résistant au chloramphénicol sont sélectionnés sur boîtes LBT + chloramphénicol 12,5 µg/mL. Un de ces clones, contenant un plasmide de 4 kb dénommé pDI24, est choisi.

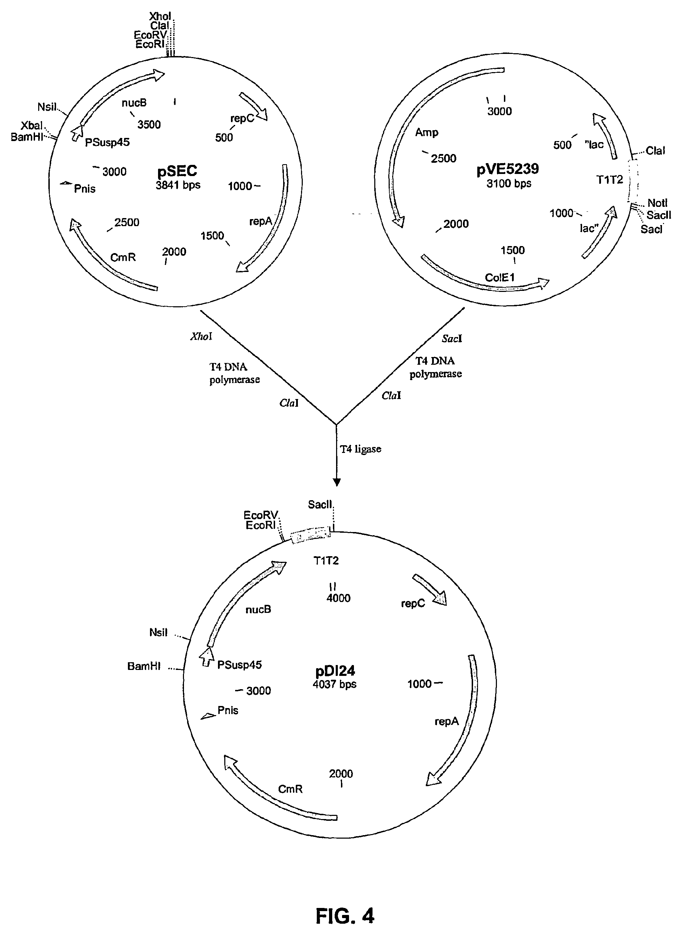

Les étapes de la construction de pDI24 sont représentées sur la Figure 4.

### Plasmide pDI1224

La fusion PSUsp45-NucB est placée sous le contrôle du système d'expression P_{Zn}*-zitR* dans le plasmide pDI12, pour produire le plasmide pDI1224.

pDI12 est digéré par *Xba*I, traité par l'ADN polymérase du phage T4, puis digéré par *Bam*HI, pour obtenir une forme linéaire de 8,1 kb. En parallèle, pDI24 est digéré par *Sac*II, traité par l'ADN polymérase du phage T4, et digéré par *Bam*HI, pour obtenir un fragment de 932 pb contenant le cadre ouvert de lecture de la protéine rapporteur NucB (sous le contrôle de RBSUsp45 et de PSUsp45), et le terminateur de transcription T1T2. Ce fragment de 932 pb est purifié et ligaturé avec la forme linéaire du vecteur pDI12, et le produit de ligation utilisé pour transformer MG1363. Les transformants sont sélectionnés sur du milieu solide M17 + glucose 0,5%, + érythromycine 5µg/mL + EDTA 0,2 mM. L'EDTA permet d'induire, grâce au système p_{Zn}-*zitR,* l'expression du rapporteur, et donc d'effectuer un premier crible du phénotype Nuc⁺ des clones recombinants. Le test d'activité Nuc est effectué selon le protocole décrit par LE LOIR *et al,* (J. Bacteriol. 176, 5135-5139, 1994).

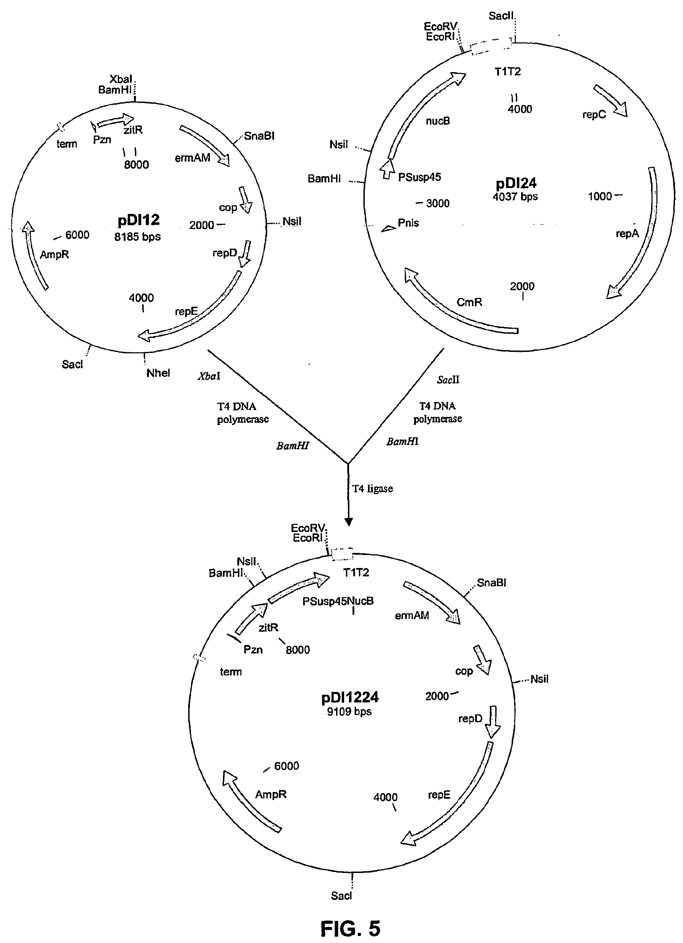

Les étapes de construction de pDI1224 sont représentées sur la Figure 5.

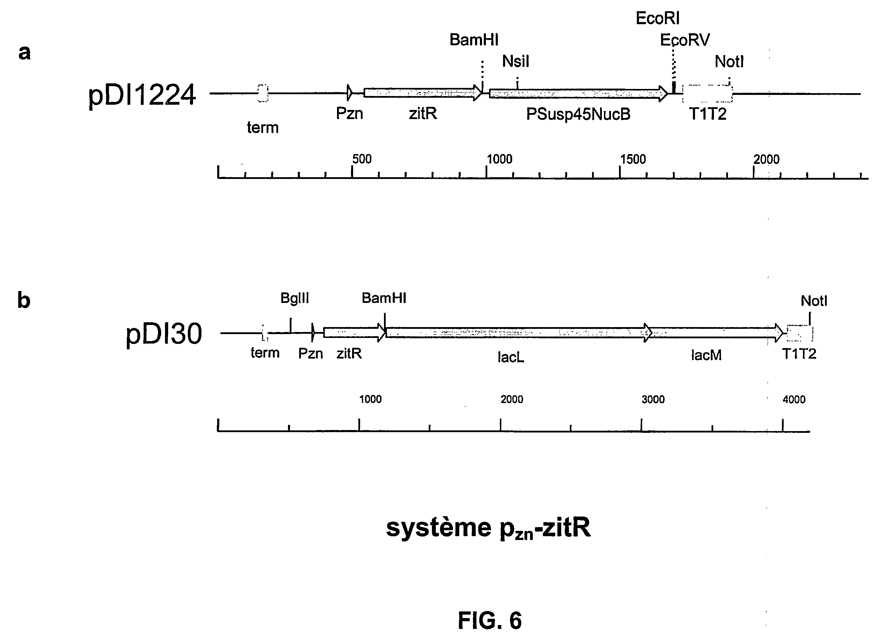

L'insert de ce plasmide contenant le gène rapporteur codant pour NucB, sous contrôle du système d'expression p_{Zn}-zitR, est schématisé sur la Figure 6a.

### Plasmide pDI30

Pour quantifier le niveau d'expression contrôlée par p_{Zn} *zitR* en fonction des conditions environnementales en Zn²⁺, un autre rapporteur de localisation cytoplasmique a été utilisé : la β-galactosidase de *Leuconostoc mesenteroides* subsp. *cremoris,* codée par l'opéron *lacLM.*

L'opéron *lacLM* a été amplifié par PCR à partir du plasmide pAMJ769 *(*MADSEN *et al,* Mol. Microbiol. 32, 75-87, 1999), en utilisant le couple d'amorces suivant :
LAC5:
5'-CGCGGATCCTTTG**AAAGGA**TATTCCTC-3' (SEQ ID NO : 15)
LAC3 :
5'-CCTACGTATTAGAAATGAATGTTAAAGC-3' (SEQ ID NO : 16).

Les amorces LAC5 et LAC3 ont été respectivement dessinées d'après la séquence du plasmide pAK80, publiée par ISRAELSEN *et al,* (Appl . Environ. Microbiol. 61, 2540-2547, 1995) et d'après la séquence des gènes *lacLM* disponible sur GeneBank sous le numéro M92281. LAC5 chevauche le site potentiel de fixation du ribosome de *lacL* (RBS, indiqué en gras sur la séquence), et LAC3 contient le codon stop de *lacM.* De plus, pour permettre le clonage du fragment PCR, on a introduit les sites de restriction *Bam*HI et *Sna*BI aux extrémités de LAC5 et LAC3 respectivement (soulignés sur les séquences). Pour la construction de pDI30, le plasmide récepteur pDI1224 a été digéré par *Bam*HI et *Eco*RV pour déléter le rapporteur sécrété PSusp45NucB, et le produit de PCR *lacLM,* après digestion par *Bam*HI et *Sna*BI*,* a été inséré à sa place. Ce mélange de ligation a été utilisé pour transformer la souche de *L. lactis* MG1363. Les transformants ont été sélectionnés en milieu gelosé M17+ glucose 0,5% + érythromycine 5µg/mL + X-Gal (5-bromo-4-chloro-3-indolyl-β-D-galactoside) 160 µg/ml + EDTA 0,5 mM. L'addition de X-Gal et d'EDTA au milieu permet de cribler les clones qui portent pDI30 pour leur phénotype bleu, lié à l'hydrolyse du X-Gal par la β-galactosidase (il a été vérifié qu'en absence d'EDTA, les clones restent blancs, ce qui indique que le milieu contient suffisamment du Zn²⁺ pour fermer l'expression de LacLM).

L'insert de ce plasmide contenant l'opéron *lacLM* codant pour la β-galactosidase, sous contrôle du système d'expression p_{Zn}-zitR, est schématisé sur la Figure 6b.

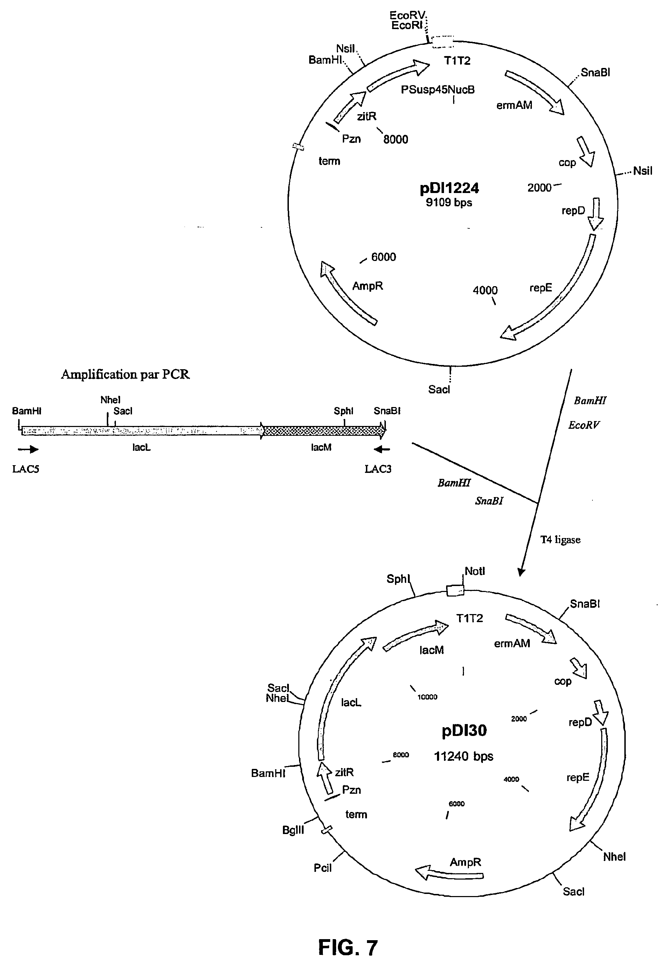

Les étapes de la construction de pDI30 sont représentées sur la Figure 7.

### EXEMPLE 3 : QUANTIFICATION DU NIVEAU D'INDUCTION D'UN GENE RAPPORTEUR DE LOCALISATION CYTOPLAMIQUE SOUS CONTROLE DU PROMOTEUR P_{ZN}ZITR

La souche de *L. lactis* MG1363 portant le plasmide pDI30 a été cultivée en milieu chimiquement défini SA, supplémenté en Zn²⁺ à 1 µM. La croissance a été effectuée pendant une nuit à 30°C sans agitation.

Le lendemain, la culture a été diluée à environ 1/100ième dans du milieu SA (qui contient du ZnSO₄ à la concentration de 10 nM) et la croissance a été suivie par mesure de la DO₆₀₀. A la valeur de DO₆₀₀~0,2, la culture a été divisée en plusieurs sous-cultures soumises à différents traitements : aucune addition ou addition d'EDTA aux concentrations de 10 - 30 - 50 - 100 - 300 ou 500 µM ou encore addition de Zn²⁺ à la concentration de 1 µM. A différents temps de traitement, la croissance a été suivie par mesure de la DO₆₀₀, et l'activité β-galactosidase (β-Gal) des extraits bactériens a été quantifiée par la méthode de Miller. En présence d'ONPG, la β-Gal produit du *O*-nitrophénol de couleur jaune que l'on peut doser par mesure de la densité optique à 420 nm ; 1 unité Miller d'activité β-Gal est définie comme produisant 1 nmol de *O*-nitrophenol par minute par unité de densité optique par mL de culture.

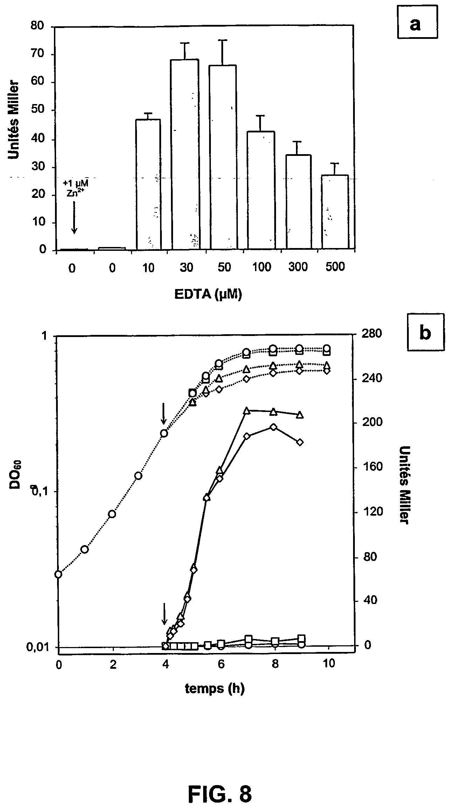

L'activité β-Gal mesurée après 1h de traitement en fonction de la concentration en EDTA rajoutée (exprimée en µM) est représentée sur la Figure 8a ; la sous-culture supplémentée en Zn²⁺ à 1µM est repérée par une flèche (+1 µM Zn)

En absence d'EDTA ou en présence de Zn²⁺, on observe un niveau d'activité β-Gal de base très faible. En présence d'EDTA, on observe une induction très nette de l'activité β-Gal qui dépend de la concentration en EDTA : le niveau d'activité maximum (induction d'un facteur >100) est obtenu pour les concentrations de 30 µM ou 50 µM d'EDTA, ce qui définit donc la gamme de concentrations optimale à utiliser dans ces conditions pour avoir une induction maximale.

Au cours de la même expérience, la croissance (courbes en pointillés) et l'activité β-Gal (courbes en traits pleins) de certaines sous-cultures ont aussi été mesurées en fonction du temps ; les résultats sont représentés sur la Figure 8b. Les sous-cultures étudiées ont subi, à la DO₆₀₀~0,2 (repérée par une flèche), les traitements suivants : aucune addition (□) ; addition d'EDTA à 30µM (△) ou 50 µM (0) ; addition de Zn²⁺ à 1 µM (O). A des intervalles de temps réguliers après ces traitements, des aliquotes sont prélevés pour quantifier l'activité β-Gal des cellules bactériennes. Le niveau d'induction dépend à la fois du temps d'exposition à l'EDTA et de sa concentration. L'induction maximale est d'un facteur >500 pour 3 à 4h en présence d'EDTA 30 µM, ce qui permet de définir les conditions d'utilisation du système.

### EXEMPLE 4: CONSTRUCTION DE PLASMIDES CONTENANT UNE SEQUENCE CODANT POUR UN PEPTIDE SIGNAL DE SECRETION

Ces plasmides sont construits par substitution d'éléments du système de sécrétion PSUsp45 du plasmide pSEC par ceux du système de sécrétion d'Exp4.

La séquence codant le peptide signal d'Exp4 (PSExp4), accompagnée des signaux de traduction d'Exp4, c'est-à-dire son RBS (ou RBSExp4) et son codon d'initiation de la traduction, a été amplifiée à partir du plasmide pVE8022 (POQUET *et al,* 1998 précité), en utilisant les couples d'amorces Exp4-5 + Exp4-NdeI et M13reverse + Exp4-NdeI, dont les séquences sont :
M13reverse:
5'-CAGGAAACAGCTATGACC-3' (SEQ ID NO: 11);
Exp4-5 :
5'-GTTCTAAGGATCCATTAACTTAAGGAG-3' (SEQ ID NO: 12);
Exp4-NdeI :
5'-TTTGTGATGCATATGCAA.ATACAACGGCTGTTG-3' (SEQ ID NO: 13);

Les amorces Exp4-5 et Exp4-NdeI ont été dessinées à partir de la partie 5' du gène *exp4* de la souche MG1363 de *L. lactis* (GENBANK numéro U95836).

Dans Exp4-5 et Exp4-NdeI, on a introduit des sites de restriction (en gras) aux extrémités de PSExp4 pour faciliter son clonage, respectivement *Bam*HI en 5' et *Nsi*I en 3' (en aval du site de clivage potentiel de PSExp4). Le site *Nsi*I est introduit à une position qui permet le clonage de PSExp4 en phase avec NucB.

Par ailleurs, Exp4-NdeI comprend un site *Nde*I (souligné) pour permettre un clonage éventuel (d'une protéine d'intérêt) en phase avec PSExp4. L'insertion des sites *NdeI* et *NsiI* n'introduit que deux acides aminés à l'extrémité N-terminale de NucB : Tyr (codé par TAT dans Exp4-NdeI) et Ala (GCA dans Exp4-NdeI), ce qui perturbe peu la séquence.

L'amplification par Exp4-5 + Exp4-NdeI à partir de l'ADN du plasmide pVE8022 produit un fragment de 117 pb.

Le peptide codé par ce fragment répond à la séquence MKKINLALLTLATLMGVSSTAVVFA↓YA (SEQ ID NO:14) qui correspond à la séquence du peptide signal d'Exp4, jusqu'au site prédit de clivage (indiqué par une flèche) suivi des deux acides aminés Y et A insérés en amont de NucB.

Après digestion par *Bam*HI et *Nsi*I le fragment Exp4-5 + Exp4-NdeI de 117 pb est inséré par ligation dans pSEC1 (CHATEL *et al,* 2001 précité) digéré par les mêmes enzymes, ce qui permet la substitution de PSUsp45 par PSExp4.

L'amplification PCR par M13reverse + Exp4-NdeI à partir de l'ADN du plasmide pVE8022 produit un fragment de 799 pb. Ce fragment contient les signaux de transcription, de traduction et de sécrétion de Exp4.

Après digestion par *Eco*RV et *Nsi*I*,* ce fragment est inséré dans pSEC1 préalablement digéré par X*ba*I traité par la polymérase du phage T4 et digéré par *Nsi*I*.* Dans le plasmide résultant, la production et la sécrétion de NucB sont ainsi placées sous contrôle des signaux de transcription, de traduction et de sécrétion de Exp4.

## Revendications

1. Procédé de construction d'une cassette d'expression d'une séquence nucléotidique d'intérêt, **caractérisé en ce que** l'on utilise pour contrôler l'expression de ladite séquence d'intérêt, un polynucléotide constitué par :
- un promoteur bactérien, dénommé ci-après p_{Zn}, contenant un site de fixation pour la protéine ZitR de *Lactococcus lactis,* lequel site comprend la séquence suivante :
AAAAATAANGTNNNNNNNTTGACATTATTTTT (SEQ ID NO:1),
dans laquelle TTGACA représente la boîte -35 dudit promoteur, et N représente A, C, G, ou T ; et
- une séquence codant pour un polypeptide présentant au moins 80% d'identité avec ZitR de *Lactococcus lactis,* placée sous contrôle transcriptionnel dudit promoteur ;
et **en ce que** l'on associe ledit polynucléotide avec au moins un site de restriction permettant l'insertion de ladite séquence d'intérêt sous contrôle transcriptionnel dudit promoteur p_{Zn}.

2. Procédé selon la revendication 1, **caractérisé en ce que** le promoteur p_{Zn} comprend la séquence suivante :
AAAAATAANGTNNNNNNNTTGACATTATTTTTNNNNNNNNNTATAAT (SEQ ID NO:2),
dans laquelle TATAAT représente la boîte -10 dudit promoteur.

3. Procédé selon la revendication 2, **caractérisé en ce que** le promoteur p_{Zn} comprend une séquence choisie parmi :
- la séquence :
AAAAATAATGTTAACTGGTTGACATTATTTTTACTTTGCTATATAATTAACCAGTA (SEQ ID NO:4) ;
- la séquence :
AAAAAATAACGTTAACTGGTTGACATTATTTTTTCTTTGCTATATAATTAACCAGTA (SEQ ID NO:5)

4. Procédé selon une quelconque des revendications 1 à 3, **caractérisé en ce que** l'on insère dans ladite cassette une séquence nucléotidique codant pour un peptide d'adressage extracellulaire, et au moins un site de restriction permettant le clonage d'une séquence nucléotidique d'intérêt en fusion traductionnelle avec ledit peptide d'adressage, sous contrôle transcriptionnel du promoteur p_{Zn}.

5. Procédé selon la revendication 4, **caractérisé en ce que** ledit peptide d'adressage extracellulaire est un peptide signal de séquence :
MKKINLALLTLATLMGVSSTAVVFA (SEQ ID NO: 6).

6. Procédé selon une quelconque des revendications 1 à 5, **caractérisé en ce que** l'on insère dans ladite cassette une séquence nucléotidique d'intérêt, sous contrôle transcriptionnel du promoteur p_{Zn}.

7. Procédé de construction d'une cassette d'expression d'une séquence nucléotidique d'intérêt, **caractérisé en ce que** l'on utilise pour contrôler l'expression de ladite séquence d'intérêt, un polynucléotide constitué par un promoteur bactérien p_{Zn}, tel que défini dans les revendications 1 à 3, et **en ce que** l'on associe ledit polynucléotide avec au moins un site de restriction permettant l'insertion de ladite séquence nucléotidique d'intérêt sous contrôle transcriptionnel dudit promoteur p_{Zn}.

8. Procédé selon la revendication 7, **caractérisé en ce que** l'on insère dans ladite cassette une séquence nucléotidique codant pour un peptide d'adressage extracellulaire, et au moins un site de restriction permettant le clonage d'une séquence nucléotidique d'intérêt en fusion traductionnelle avec ledit peptide d'adressage, sous contrôle transcriptionnel du promoteur p_{Zn},

9. Procédé selon la revendication 8, **caractérisé en ce que** ledit peptide d'adressage extracellulaire est un peptide signal de séquence SEQ ID NO: 6.

10. Procédé selon une quelconque des revendications 7 à 9, **caractérisé en ce que** l'on insère dans ladite cassette une séquence nucléotidique d'intérêt, sous contrôle transcriptionnel du promoteur p_{Zn}.

11. Procédé de construction d'un vecteur recombinant, **caractérisé en ce qu'**il comprend la construction d'une cassette d'expression par un procédé selon une quelconque des revendications 1 à 10, et l'insertion de ladite cassette dans un vecteur.

12. Procédé de transformation d'une bactérie à gram-positif, **caractérisé en ce qu'**il comprend la construction d'une cassette d'expression par un procédé selon une quelconque des revendications 1 à 10, et la transformation de ladite bactérie par ladite cassette d'expression.

13. Procédé selon la revendication 12, **caractérisé en ce que** ladite bactérie est une bactérie lactique.

14. Utilisation d'une cassette d'expression susceptible d'être obtenue par un procédé selon une quelconque des revendications 1 à 10, ou d'un vecteur recombinant susceptible d'être obtenu par un procédé selon la revendication 11, pour la production d'une protéine d'intérêt dans une bactérie à gram-positif.

15. Procédé de production d'une protéine d'intérêt dans une bactérie à gram-positif, **caractérisé en ce qu'**il comprend:
- l'introduction dans ladite bactérie d'une cassette d'expression susceptible d'être obtenue par un procédé selon une quelconque des revendications 1 à 6, comprenant une séquence codant pour ladite protéine d'intérêt, placée sous contrôle transcriptionnel du promoteur p_{Zn} ;
- la culture de ladite bactérie dans un milieu contenant une quantité de Zn²⁺ suffisante pour réprimer l'expression de ladite protéine ;
- l'induction de la production de ladite protéine par déplétion en Zn²⁺dudit milieu ;
- la récupération de la protéine produite.

16. Procédé selon la revendication 15, **caractérisé en ce que** la déplétion en Zn²⁺ dudit milieu est obtenue par addition d'un chélateur des cations divalents.

17. Procédé selon la revendication 15, **caractérisé en ce que** la déplétion en Zn²⁺ dudit milieu est obtenue par culture de la bactérie jusqu'à épuisement du Zn²⁺ initialement présent dans le milieu.

18. Procédé de production d'une protéine d'intérêt dans une bactérie à gram-positif dans laquelle le répresseur ZitR endogène est inactif, **caractérisé en ce qu'**il comprend :
- l'introduction dans ladite bactérie d'une cassette d'expression susceptible d'être obtenue par un procédé selon une quelconque des revendications 7 à 10, comprenant une séquence codant pour ladite protéine d'intérêt, placée sous contrôle transcriptionnel du promoteur p_{Zn} ;
- la culture de ladite bactérie ;
- la récupération de la protéine produite.

## Claims

1. Process for constructing an expression cassette for a nucleotide sequence of interest, **characterised in that** the expression of said sequence of interest is controlled by a polynucleotide consisting of:
- a bacterial promoter, hereinafter designated p_{Zn}, containing a fixing site for the protein ZitR of *Lactococcus lactis,* said site comprising the following sequence:
AAAAATAANGTNNNIVNNNTTGACATTATTTTT (SEQ ID NO:1),
wherein TTGACA represents the -35 box of said promoter, and N represents A, C, G or T; and
- a sequence coding for a polypeptide having at least 80% identity with the protein ZitR of *Lactococcus lactis,* placed under the transcription control of said promoter;
and **in that** said polynucleotide is combined with at least one restriction site allowing said sequence of interest to be inserted under the transcription control of said promoter p_{Zn}.

2. Process according to claim 1, **characterised in that** the promoter p_{Zn} comprises the following sequence:
AAAAATAANGTNNNNNNNTTGACATTATTTTTNNNNNNNNNTA TAAT (SEQ ID NO:2),
wherein TATAAT represents the -10 box of said promoter.

3. Process according to claim 2, **characterised in that** the promoter p_{Zn} comprises a sequence selected from among:
- the sequence:
AAAAATAATGTTAACTGGTTGACATTATTTTTACTTTGCTATATA ATTAACCAGTA (SEQ ID NO:4);
- the sequence:
AAAAAATAACGTTAACTGGTTGACATTATTTTTTCTTTGCTATAT AATTAACCAGTA (SEQ ID NO:5).

4. Process according to any one of claims 1 to 3, **characterised in that** there is inserted into said cassette a nucleotide sequence coding for an extracellular addressing peptide and at least one restriction site allowing the cloning of a nucleotide sequence of interest in translational fusion with said addressing peptide, under the transcriptional control of the promoter p_{Zn}.

5. Process according to claim 4, **characterised in that** said extracellular addressing peptide is a signal peptide of the sequence:
MKKINLALLTLATLMGVSSTAVVFA (SEQ ID NO:6).

6. Process according to any one of claims 1 to 5, **characterised in that** there is inserted into said cassette a nucleotide sequence of interest under the transcriptional control of the promoter p_{Zn}.

7. Process for constructing an expression cassette for a nucleotide sequence of interest, **characterised in that** the expression of said sequence of interest is controlled by means of a polynucleotide consisting of a bacterial promoter p_{Zn}, as defined in claims 1 to 3, and **in that** said polynucleotide is combined with at least one restriction site allowing the insertion of said nucleotide sequence of interest under the transcriptional control of said promoter p_{Zn}.

8. Process according to claim 7, **characterised in that** there is inserted into said cassette a nucleotide sequence coding an extracellular addressing peptide and at least one restriction site allowing the cloning of a nucleotide sequence of interest in translational fusion with said addressing peptide, under the transcriptional control of the promoter p_{Zn}.

9. Process according to claim 8, **characterised in that** said extracellular addressing peptide is a signal peptide of sequence SEQ ID NO:6.

10. Process according to any one of claims 7 to 9, **characterised in that** a nucleotide sequence of interest is inserted into said cassette, under the transcriptional control of the promoter p_{Zn}.

11. Process for constructing a recombinant vector, **characterised in that** it comprises constructing an expression cassette by a process according to any one of claims 1 to 10 and inserting said cassette in a vector.

12. Process for transforming a gram-positive bacterium, **characterised in that** it comprises constructing an expression cassette by a process according to any one of claims 1 to 10 and transforming said bacterium using said expression cassette.

13. Process according to claim 12, **characterised in that** said bacterium is a lactic bacterium.

14. Use of an expression cassette which may be obtained by a process according to any one of claims 1 to 10, or a recombinant vector which may be obtained by a process according to claim 11, for producing a protein of interest in a gram-positive bacterium.

15. Process for producing a protein of interest in a gram-positive bacterium, **characterised in that** it comprises:
- inserting into said bacterium an expression cassette which may be obtained by a process according to any one of claims 1 to 6, comprising a sequence coding for said protein of interest, placed under the transcriptional control of the promoter p_{Zn};
- cultivating said bacterium in a medium containing a quantity of Zn²⁺ sufficient to suppress the expression of said protein;
- inducing the production of said protein by depleting the medium of Zn²⁺;
- recovering the protein produced.

16. Process according to claim 15, **characterised in that** the Zn²⁺ depletion of said medium is obtained by adding a divalent cation chelating agent.

17. Process according to claim 15, **characterised in that** the Zn²⁺ depletion of said medium is achieved by cultivating the bacteria until the Zn²⁺ initially present in the medium has been exhausted.

18. Process for producing a protein of interest in a gram-positive bacterium in which the endogenous ZitR repressor is inactive, **characterised in that** it comprises:
- introducing into said bacterium an expression cassette which may be obtained by a process according to any one of claims 7 to 10, comprising a sequence coding for said protein of interest, placed under the transcriptional control of the promoter p_{Zn},
- cultivating said bacterium;
- recovering the protein produced.

## Patentansprüche

1. Verfahren zur Herstellung einer Expressionskassette für eine Nukleotidsequenz von Interesse, **dadurch gekennzeichnet, dass** zur Kontrolle der Expression der besagten Sequenz von Interesse ein Polynukleotid verwendet wird, das gebildet ist aus:
- einem bakteriellen Promotor, nachfolgend p_{Zn} genannt, enthaltend eine Bindungsstelle für das Protein ZitR aus *Lactococcus lactis,* wobei die besagte Bindungstelle die folgende Sequenz enthält:
AAAAATAANGTNNNNNNNTTGACATTATTTTT (SEQ ID NO: 1),
wobei TTGACA die -35-Region des besagten Promotors repräsentiert und wobei N A, C, G, oder T repräsentiert; und
- eine Sequenz, die für ein Polypeptid codiert, das eine Identität von mindestens 80% mit dem Protein ZitR aus *Lactococcus lactis* aufweist und unter transkriptioneller Kontrolle des besagten Promotors steht;
und wobei das besagte Polynukleotid mit mindestens einer Restriktionsschnittstelle ausgestattet ist, die es erlaubt die besagte Sequenz von Interesse unter der transkriptionellen Kontrolle des besagten Promotors p_{Zn} zu inserieren.

2. Das Verfahren nach Anspruch 1, **dadurch gekennzeichnet, dass** der Promotor p_{Zn} die folgende Sequenz enthält:
AAAAATAANGTNNNNNNNTTGACATTATTTTTNNNNNNNNNTATAAT (SEQ ID NO. 2), wobei die Sequenz TATAAT die -10-Region des Promotors darstellt.

3. Das Verfahren nach Anspruch 2, **dadurch gekennzeichnet, dass** der Promotor p_{Zn} eine Sequenz aufweist, die ausgewählt ist aus der Gruppe:
- der Sequenz:
AAAAATAATGTTAACTGGTTGACATTATTTTTACTTTGCTATATAA TTAACCAGTA (SEQ ID NO: 4);
- der Sequenz :
AAAAAATAACGTTAACTGGTTGACATTATTTTTTCTTTGCTATATA ATTAACCAGTA (SEQ ID NO: 5).

4. Verfahren nach irgendeinem der Ansprüche 1 bis 3, **dadurch gekennzeichnet, dass** in diese besagte Kassette eine Nukleotidsequenz, die für ein Peptid für den extrazellulären Export codiert, und mindestens eine Restriktionsschnittstelle inseriert wird, die es erlaubt, eine Nukleotidsequenz von Interesse in translationeller Fusion mit dem besagten Peptid zum extrazellulären Export unter der transkriptionellen Kontrolle des Promotors p_{Zn} zu klonieren.

5. Verfahren nach Anspruch 4, **dadurch gekennzeichnet, dass** das besagte Peptid zum extrazellulären Transport ein Signalpeptid mit der Sequenz MKKINLALLTLATLMGVSSTAVVFA (SEQ ID NO: 6) ist.

6. Verfahren nach irgendeinem der Ansprüche 1 bis 5, **dadurch gekennzeichnet, dass** in die besagte Kassette eine Nukleotidsequenz von Interesse unter transkriptioneller Kontrolle des Promotors p_{Zn} inseriert wird.

7. Verfahren zur Herstellung einer Expressionskassette für eine Nukleotidsequenz von Interesse, **dadurch gekennzeichnet, dass** zur Kontrolle der Expression der Sequenz von Interesse ein Polynukleotid verwendet wird, das aus einem bakteriellen Promoter p_{Zn} gebildet ist, der gemäß den Ansprüche 1 bis 3 definiert ist, und dass das besagte Polynukleotid mit mindestens einer Restriktionsschnittstelle ausgestattet ist, die die Insertion der besagten Nukleotidsequenz von Interesse unter der transkriptionellen Kontrolle des genannten Promoters p_{Zn} erlaubt.

8. Verfahren nach Anspruch 7, **dadurch gekennzeichnet, dass** in die besagte Kassette eine Nukleotidsequenz, die für ein Peptid zum extrazellulären Transport codiert, und mindestens eine Restriktionsschnittstelle inseriert wird, die die Klonierung einer Nukleotidsequenz von Interesse in translationeller Fusion mit dem besagten Peptid zum extrazellulären Transport erlaubt, unter der transkriptionellen Kontrolle des Promotors p_{Zn}.

9. Verfahren nach Anspruch 8, **dadurch gekennzeichnet, dass** das besagte Peptid zum extrazellulären Transport ein Signalpeptid mit der Sequenz SEQ ID NO: 6 ist.

10. Verfahren nach irgendeinem der Ansprüche 7 bis 9, **dadurch gekennzeichnet, dass** in die besagte Kassette eine Nukleotidsequenz von Interesse inseriert wird unter der transkriptionellen Kontrolle des Promotors p_{Zn}.

11. Verfahren zur Herstellung eines rekombinanten Vektors, **dadurch gekennzeichnet, dass** es die Herstellung einer Expressionskassette nach einem Verfahren gemäß irgendeinem der Ansprüche 1 bis 10 umfasst, sowie die Insertion der genannten Kassette in einen Vektor.

12. Verfahren zur Transformation eines grampositiven Bakteriums, **dadurch gekennzeichnet, dass** es die Herstellung einer Expressionskassette nach einem Verfahren gemäß irgendeinem der Ansprüche 1 bis 10 umfasst, und die Transformation der genannten Bakterien mit der besagten Expressionskassette.

13. Verfahren nach Anspruch 12, **dadurch gekennzeichnet, dass** die Bakterien Milchsäurebakterien sind.

14. Verwendung einer Expressionskassette, erhältlich nach einem Verfahren gemäß irgendeinem der Ansprüche 1 bis 10, oder eines rekombinanten Vektors, erhältlich nach einem Verfahren gemäß Anspruch 11 für die Herstellung eines Proteins von Interesse in einem grampositiven Bakterium.

15. Verfahren zur Herstellung eines Proteins von Interesse in einem grampositiven Bakterium, **dadurch gekennzeichnet, dass** es umfasst:
- die Einführung einer Expressionskassette erhältlich nach einem Verfahren gemäß irgendeinem der Ansprüche 1 bis 6 in das besagte Bakterium, wobei die Expressionskassette eine Sequenz enthält, die für das Protein von Interesse codiert und unter der transkriptionellen Kontrolle des Promoters p_{Zn} angeordnet ist;
- Kultivierung des Bakteriums in einem Medium enthaltend eine Menge Zn²⁺, die ausreichend ist zur Unterdrückung der Expression des genannten Proteins;
- die Induktion der Produktion des genannten Proteins durch Entfernen des Zn²⁺ aus dem genannten Medium;
- die Gewinnung des hergestellten Proteins.

16. Verfahren nach Anspruch 15, **dadurch gekennzeichnet, dass** die Entfernung von Zn²⁺ aus dem genannten Medium durch die Zugabe eines Chelatierungsmittels für zweiwertige Kationen erreicht wird.

17. Verfahren nach Anspruch 15, **dadurch gekennzeichnet, dass** die Entfernung von Zn²⁺ aus dem genannten Medium durch Kultivierung der Bakterien bis zur Erschöpfung des anfänglich in dem Medium enthaltenen Zn²⁺ erreicht wird.

18. Verfahren zur Herstellung eines Proteins von Interesse in einem grampositiven Bakterium, in dem der endogene Repressor ZitR inaktiv ist, **dadurch gekennzeichnet, dass** es umfasst:
- die Einführung einer Expressionskassette erhältlich nach einem Verfahren gemäß irgendeinem der Ansprüche 7 bis 10 in das besagte Bakterium, wobei die Expressionskassette eine Sequenz enthält, die für das Protein von Interesse codiert und unter der transkriptionellen Kontrolle des Promotors p_{Zn} angeordnet ist;
- Kultivierung der Bakterien;
- Gewinnung des hergestellten Proteins.
